# FASCICULE DE BREVET EUROPEEN

(11) **EP 1 397 122 B1**
(45) Date de publication et mention de la délivrance du brevet: **10.12.2008**
(21) Numéro de dépôt: 02740821.0
(22) Date de dépôt: 27.05.2002
(51) Int. Cl.: A61K 9/16

(54) **ADJUVANT D'IMMUNITÉ SOUS FORME SOLIDE ET VACCIN LE CONTENANT**
FESTES IMMUNITÄTADJUVANS UND DIESES ENTHALTENDE VAKZINE
SOLID IMMUNITY ADJUVANT AND VACCINE CONTAINING THE SAME

(30) Priorité: 31.05.2001 FR 0107149
(43) Date de publication de la demande: 17.03.2004
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: DUPUIS, Laurent, F-94800 Villejuif (FR); GANNE, Vincent, F-75321 PARIS cedex 07 (FR); AUCOUTURIER, Jérôme, F-92290 Châtenay-Malabry (FR); TROUVE, Gérard, F-81100 Castres (FR)
(74) Mandataire: Conan, Philippe Claude
(86) Numéro de dépôt international: PCT/FR2002/001775
(87) Numéro de publication internationale: WO 2002/096386

(56) Documents cités:
- EP-A- 1 095 662
- WO-A-94/15636
- WO-A-98/41188
- GB-A- 1 379 008

## Description

La présente invention concerne de nouveaux adjuvants pour compositions vaccinales ainsi que leur utilisation pour préparer des compositions vaccinales injectables, lesdites compositions comprenant au moins un antigène, notamment un antigène d'origine virale, bactérienne ou parasitaire et au moins un adjuvant.

De très nombreuses substances sont décrites comme améliorant la réponse immunitaire à un antigène.

Il y a les sels minéraux insolubles dans l'eau, parmi lesquels l'hydroxyde d'aluminium et le phosphate de calcium sont les plus connus et sont les seuls autorisés à ce jour pour la vaccination humaine. Ils induisent peu de réactions d'intolérance au site d'injection mais leur efficacité est par contre médiocre et leur effet de courte durée. Ils se présentent sous forme de gels.

Il y aussi les huiles injectables utilisées comme adjuvants dans les vaccins vétérinaires. Elles sont très efficaces mais elles induisent parfois des réactions locales. Elles sont mises en oeuvre en mélange avec le milieu antigénique pour former des émulsions fluides injectables.

Lorsque ces émulsions sont du type huile - dans - eau (H/E), la protection de l'animal contre la maladie est assurée rapidement, mais seulement pendant une courte durée, de l'ordre de quelques mois.

Lorsque ces émulsions sont du type eau - dans - huile (E/H), la protection de l'animal contre la maladie n'est assurée qu'au bout de quelques semaines mais elle dure longtemps, jusqu'à une année ou plus. On pense que cette protection à long terme est due à l'enrobage par l'huile, des gouttes de milieu antigénique.

Il y a encore les sels hydrosolubles de cations multivalents associés à un anion organique qui ont été décrits dans les brevets français publiés sous les numéros FR 2 733 151 et FR 2 754 715. Ces sels solubles sont très bien tolérés et assurent une protection rapide, mais de courte durée, lorsqu'ils sont utilisés comme adjuvant unique. Lorsqu'ils sont combinés à des huiles sous forme d'émulsions ou de micro - émulsions (H/E), ils induisent une protection prolongée de durée cependant inférieure à celle conférée par des vaccins de type (E / H).

WO 94 15636 et WO 98 41188 divulguent des microsphères d'immunogènes adsorbées sur un sel d'aluminium ou incluses dans un sucre. Cependant, de telles formulations solides sont délicates à préparer, les procédés comprenant le passage par une phase liquide, voire une émulsion puis un séchage.

Il n'existe pas aujourd'hui de composition adjuvante solide qui puisse être dispersée aisément dans une phase aqueuse par simple agitation.

C'est pourquoi l'invention a pour objet, une composition solide adjuvante de vaccin caractérisée en ce qu'elle comprend un support solide injectable.

Par composition solide, on désigne plus particulièrement une composition qui se présente sous la forme de granulés.

Par support solide injectable, on désigne un composé solide sur lequel un liquide est capable d'être absorbé, adsorbé, imprégné ou ancré tout en conduisant à un mélange résultant solide, sans lui donner un aspect pâteux ou fluide.

Les poudres ou granulés objet de la présente invention contiennent généralement au plus 5% en poids d'eau, plus particulièrement au plus 4% en poids d'eau et tout particulièrement au plus 3% en poids d'eau.

Comme exemples de support solide injectable contenu dans la composition objet de la présente invention, il y a par exemple une silice pyrogénée hydrophile comme l'AEROSIL^{™} 200, un sucre injectable et plus particulièrement le lactose, le dextrose ou le mannitol, un polysaccharide et plus particulièrement une dextrine ou une cyclodextrine, un polymère cellulosique et plus particulièrement l'hydroxypropyl méthyl cellulose, la méthyl cellulose ou l'éthyl cellulose, un sel de cation métallique multivalent ou un mélange de sels de cations métalliques multivalents ou un mélange de deux ou plusieurs de ces composés.

Comme exemples de cations métalliques multivalents, il y a plus particulièrement les cations métalliques divalents ou trivalents et tout particulièrement les cations divalents du calcium, du magnésium, du manganèse ou du zinc ou bien les cations trivalents du fer ou de l'aluminium.

Les sels de cations métalliques sont injectables. Il s'agit par exemple d'un hydroxyde, d'un carbonate, d'un citrate, d'un gluconate, d'un glucoheptonate, d'un fructoheptonate, d'un lactate, d'un acétate, d'un propionate, d'un salicylate, d'un chlorure ou d'un glycérophosphate.

Comme exemples de sels mis en oeuvre dans la préparation de la composition objet de la présente invention, il y a l'hydroxyde de calcium, le carbonate de magnésium, le carbonate de manganèse, le gluconate de calcium, le gluconate de manganèse, le glycérophosphate de manganèse, le gluconate de zinc, le fructoheptonate de calcium, le salicylate d'aluminium ou l'acétate d'aluminium.

Selon un premier aspect particulier de la présente invention, le support solide injectable est un sel de cation métallique multivalent ou un mélange de sels de cations métalliques multivalents et est plus particulièrement un sel de calcium ou un sel de manganèse ou un mélange de ces sels.

Selon un deuxième aspect particulier de la présente invention, le support solide injectable est un mélange d'un ou plusieurs sels de cations métalliques multivalents avec un ou plusieurs sucres injectables.

Selon un troisième aspect particulier de la présente invention, le support solide injectable est de la silice pyrogénée hydrophile comme l'AEROSIL^{™} 200 ou un mélange d'AEROSIL^{™} 200 avec un ou plusieurs sucres injectables.

Selon un quatrième aspect particulier de la présente invention, le support solide injectable est un mélange d'un ou plusieurs sels de cations métalliques multivalents et de silice pyrogénée hydrophile.

La composition telle que définie précédemment peut comprendre en outre un agent tensioactif ou un mélange d'agents tensioactifs.

Cet agent tensioactif ou ce mélange d'agents tensioactifs a de préférence un nombre HLB global compris entre 5 et 15. Au sens de la présente invention, le nombre HLB est calculé par la formule : HLB = 20 (1-Iₛ/Iₐ), dans laquelle Iₛ représente l'indice de saponification et Iₐ, l'indice d'acide dudit tensioactif ou dudit mélange d'agents tensioactifs. Ces deux indices, de saponification et d'acide, sont déterminés par des méthodes décrites dans la Pharmacopée européenne.

Le ou les agents tensioactifs sont principalement choisis parmi les corps gras modifiés et de préférence parmi les corps gras modifiés ayant un nombre HLB global compris entre 6 et 14.

Les corps gras modifiés utilisés de la présente invention, peuvent être d'origine minérale végétale ou animale. Comme corps gras modifiés d'origine minérale, il y a les huiles d'origine pétrolière. Comme corps gras modifiés d'origine végétale, il y a les huiles végétales modifiées, par exemple les huiles modifiées d'arachide, d'olive, de sésame, de soja, de germes de blé, de pépins de raisin de tournesol, de ricin, de lin, de soja, de maïs, de coprah, de palme, de noix, de noisettes ou de colza. Comme corps gras modifiés d'origine animale, il y a par exemple le squalane modifié, le squalène modifié, l'huile modifiée de spermaceti ou l'huile modifiée de suif.

Par corps gras modifiés, on désigne notamment les dérivés alcoxylés de corps gras et plus particulièrement les dérivés alcoxylés d'huiles ou les dérivés alcoxylés d'esters alkyliques d'huiles et plus particulièrement, les dérivés éthoxylés et/ou propoxylés d'huiles ou les dérivés éthoxylés et/ou propoxylés des esters méthylique, éthylique, propyliques linéaire ou ramifié ou butyliques, linéaire ou ramifiés, desdites huiles. L'invention a plus spécifiquement pour objet une composition telle que définie précédemment, dans laquelle le corps gras modifié est choisi parmi les dérivés éthoxylés d'huiles ayant un nombre d'OE, compris entre 1 et 60. L'invention a particulièrement pour objet, une composition telle que définie précédemment, dans laquelle le corps gras modifié est un dérivé alcoxylés d'huile de maïs ou un mélange de dérivés alcoxylés d'huile de maïs, ayant un nombre HLB global compris entre 10 et 14 ou une composition telle que définie précédemment, dans laquelle le corps gras modifié est un dérivé éthoxylé d'huile de ricin ou un mélange de dérivés alcoxylés d'huile de ricin, ayant un nombre HLB global compris entre 7 et 10. Comme exemples de telles compositions, il y a la composition dans laquelle le corps gras modifié est choisi parmi les dérivés éthoxylés d'huile de maïs ayant un nombre d'OE compris entre 20 et 40 ou la composition dans laquelle le corps gras modifié est un mélange de dérivés éthoxylés d'huile de ricin ayant un nombre d'OE égal à 7 et de dérivés éthoxylés d'huile de ricin ayant un nombre d'OE égal à 60.

Selon un cinquième aspect particulier de la présente invention, celle-ci a pour objet une composition telle que définie précédemment, dans laquelle le ou les agents tensioactifs sont choisis parmi les dérivés alcoxylés d'esters d'acides gras et de polyols ou les dérivés alcoxylés d'éthers d'alcools gras et de polyols et plus particulièrement, les triglycérides d'acides gras alcoxylés, les esters alcoxylés de polyglycérol d'acides gras, les dérivés alcoxylés d'esters d'acides gras avec un hexol, tel que par exemple le sorbitol ou le mannitol ou les dérivés alcoxylés d'esters d'acides gras avec un anhydride d'hexol, comme le sorbitane ou le mannitane.

Comme acides gras appropriés à la préparation de ces esters modifiés il y a plus particulièrement, ceux comportant de 12 à 22 atomes de carbone, avantageusement un acide gras liquide à 20°C, tels que par exemple, ceux comportant de 16 à 18 atomes de carbone, comme l'acide oléique, l'acide ricinoléique ou l'acide isostéarique.

La composition, telle que définie ci-dessus, contient notamment, un ou plusieurs dérivés éthoxylés d'esters d'acides gras et de polyol ou dérivés éthoxylés d'éthers d'alcools gras et de polyols, ayant un nombre d'OE, compris entre 1 et 60. L'agent tensioactif ou le mélange d'agents tensioactifs de cette composition telle que définie ci-dessus, a plus particulièrement un nombre HLB global compris entre 10 et 14 et de préférence entre 12 et 13,5.

La composition telle que définie précédemment peut aussi comprendre un ou plusieurs corps gras.

Par corps gras, on entend dans le cadre de la présente invention, un composé ou un mélange de composés choisis les huiles d'origine minérale, végétale ou animale, les esters alkyliques desdites huiles, les esters alkyliques d'acides gras ou les éthers alkyliques d'acides gras, les esters d'acides gras et de polyols ou les éthers d'alcools gras et de polyols. Comme exemples d'huile d'origine minérale, il y a les huiles d'origine pétrolière, comme les huiles blanches minérales telles que le MARCOL^{™} 52. Comme exemples d'huiles d'origine végétale, il y a l'huile d'arachide, l'huile d'olive, l'huile de sésame, l'huile de soja, l'huile de germes de blé, l'huile de pépins de raisin, l'huile de tournesol, l'huile de ricin, l'huile de lin, l'huile de soja, l'huile de maïs, l'huile de coprah, l'huile de palme, l'huile de noix, l'huile de noisette, l'huile de colza ou encore le squalane ou le squalène d'olive. Comme exemples d'huiles d'origine animale, il y a l'huile de spermaceti, l'huile de suif, le squalane ou le squalène extraits des foies de poissons. Comme exemples d'esters alkyliques d'huiles, il y a les esters méthyliques, éthylique, propyliques linéaire ou ramifié ou butyliques, linéaire ou ramifiés, desdites huiles. Comme acides gras appropriés à la préparation des esters cités ci-dessus, il y a plus particulièrement, ceux comportant de 12 à 22 atomes de carbone, tels que par exemple, l'acide myristique, l'acide palmitique, l'acide oléique, l'acide ricinoléique ou l'acide isostéarique et avantageusement un acide gras liquide à 20°C. Comme exemples d'esters d'acides gras, il y a les esters alkyliques d'acides gras, tels que, l'oléate d'éthyle, l'oléate de méthyle, le myristate d'isopropyle ou le palmitate d'octyle, les esters d'acides gras et de polyols, et plus particulièrement, les monoglycérides d'acides gras, les diglycérides d'acides gras, les triglycérides d'acides gras, les esters d'acides gras avec un polyglycérol ou les esters d'acides gras et de propylèneglycol, les esters d'acides gras avec un hexol, tel que par exemple le sorbitol ou le mannitol, les esters d'acides gras avec un anhydride d'hexol, comme le sorbitane ou le mannitane.

Au sein d'un même produit commercial, l'huile est parfois associée avec un ou plusieurs agents tensioactifs non ioniques. Comme exemples d'agents tensioactifs que l'on peut associer à une huile dans une même phase adjuvante, il y a ceux décrits précédemment, comme ayant intrinsèquement des propriétés adjuvantes. De façon plus générale, il y a les tensioactifs non ioniques des familles des esters ou éthers d'acides gras et de sucre comme le sorbitol, le mannitol, le saccharose ou le glucose, les esters d'acides gras et de glycérol ou de polyol, des dérivés hydrophiles de ces esters obtenus par greffage de fonctions alcool, éther - oxyde, carboxylique, amine ou amide, des lécithines, des acides ou alcools gras éthoxylés et ou propoxylés, des chaînes grasses de ces tensioactifs comportant de 8 à 22 atomes de carbone. Parmi ces agents tensioactifs, on préfère ceux ayant une chaîne grasse comportant de 14 à 20 atomes de carbone et plus particulièrement, les acides oléique, ricinoléique, cétostéarique et leurs dérivés et tout particulièrement les oléates de mannitol et les dérivés de oléates de mannitol obtenus par greffage de fonctions hydrophiles telles que par exemple les fonctions amide, amine, alcool, polyol, carboxylique ou de radicaux éthoxy, propoxy et/ou butoxy ou les oléates de mannitane ou leurs dérivés ; ils sont obtenus par anhydrisation de la chaîne carbonée polyhydroxylée du mannitol qui se cyclise alors en 1 - 4 ou en 2 - 6. De façon générale lorsqu'un agent tensioactif non ionique ou un mélange d'agents tensioactifs non ioniques est présent dans une composition vaccinale, associé à une huile pour exprimer ses propriétés émulsionnantes, sa concentration est comprise entre 0,01 mg / ml et 500 mg / ml et de préférence entre 0,1 mg / ml et 200 mg / ml. Comme exemples d'association d'huiles avec un agent tensioactif non ionique, il y a les produits commercialisés sous le nom MONTANIDE^{™}, dont les caractéristiques sont exposées dans le tableau suivant :

| Nom | Composition | Emulsion produite après dispersion dans l'eau (type ; % de phase aqueuse en poids) | Conductivité - à 25°C (µS.cm⁻¹) | Viscosité (mPa.s) |
|---|---|---|---|---|
| MONTANIDE^{™} ISA 25 | Huile minérale + esters de mannitol | H/E 75 % | 5000 | 20 |
| MONTANIDE^{™} ISA 25A | Huile minérale + esters de mannitol + avridine | H/E 75 % | 5000 | 20 |
| MONTANIDE^{™} ISA 28 | Huile minérale + esters de mannitol + oléate d'éthyle | H/E 75 % | 1000 | 25 |
| MONTANIDE^{™} ISA 206 | Huile minérale + esters de mannitol + PEG 500* | E/H/E 50 % | 1000 | 50 |
| MONTANIDE^{™} ISA 50 | Huile minérale + esters de mannitol | E/H 50 % | 1 | 200 |
| MONTANIDE^{™} ISA 708 | Huile végétale + esters de mannitol | E/H 30 % | 1 | 70 |

| | | | | |
|---|---|---|---|---|
| * PEG : polyéthylèneglycol | | | | |

selon un aspect particulier de la présente invention, elle comprend une association comprenant :
de 50 % à 95 % en poids d'oléate de mannitane éthoxylé ayant un nombre d'OE compris entre 5 et 15, et, de préférence, entre 7 et 11 et
de 5 % à 50% en poids d'oléate de mannitane.
La composition selon l'invention, peut comporter un agent stimulant immunitaire conventionnel tel l'Avridine®, la N,N-dioctadecyl - N',N'-bis(2-hydroxyéthyl) propane-diamine, les dérivés du MDP (muramyl dipeptide), notamment le thréonyl-MDP, les dérivés de l'acide mycolique ou les dérivés du Lipide A.

La composition telle que définie précédemment, comprend généralement entre 10 % et 90 % en poids de support solide injectable, et entre 10 % et 90 % en poids de tensioactif ou entre 10 % et 90% en poids d'un mélange constitué d'un ou plusieurs corps gras et d'un ou plusieurs tensioactifs.

Selon un sixième aspect particulier de la présente invention, la composition telle que définie précédemment, comprend au plus 30 % en poids de support solide injectable, lorsque ce support solide injectable est de la silice pyrogénée hydrophile comme l'AEROSIL^{™} 200, un mélange de silice pyrogénée hydrophile avec un ou plusieurs sucres injectables ou un mélange d'un ou plusieurs sels de cations métalliques multivalents et de silice pyrogénée hydrophile.

Selon un septième aspect particulier de la présente invention, la composition telle que définie précédemment, comprend au moins 50% en poids de support solide injectable, lorsque ce support solide injectable est un sel de cation métallique multivalent ou un mélange de sels de cations métalliques multivalents ou un mélange d'un ou plusieurs sels de cations métalliques multivalents avec un ou plusieurs sucres injectables.

La composition selon l'invention peut aussi comprendre entre 1% et 10% en poids d'agent dispersant injectable.

Comme agent dispersant injectable, il y a par exemple les sels de sodium ou d'ammonium des acides citrique, glycolique, lactique, phosphorique, tartrique ou acétique.

La composition solide, objet de la présente invention est formulée sous forme de granulés.

L'invention a aussi pour objet un procédé de préparation de la composition telle que définie précédemment, comprenant les étapes suivantes :
(a) si nécessaire, la préparation d'un mélange de tous les éléments constitutifs du support solide injectable avec ajout éventuel de l'agent dispersant,
(b) l'addition sous agitation, des tensioactifs, et éventuellement des corps gras liquides, au mélange préparé à l'étape (a), ou à la poudre de support solide injectable, dans un mélangeur pour former une poudre soit dans un mélangeur - granulateur pour former des granulés.

Selon un autre aspect de la présente invention, celle - ci a pour objet, l'utilisation de la composition telle que définie précédemment, comme phase adjuvante d'une composition vaccinale. Le vaccin ainsi formé est destiné à la vaccination humaine ou vétérinaire. -

L'invention a aussi pour objet un procédé de préparation d'une composition injectable comprenant l'adjonction à une phase antigénique comme adjuvant d'immunité, d'une quantité efficace de la composition telle que définie précédemment.

La phase antigénique contient un ou plusieurs antigène ou générateurs in vivo de composés comprenant une séquence d'acides aminés.

Par antigène ou au moins un générateur in vivo d'un composé comprenant une séquence d'acides aminés, on désigne soit des micro-organismes tués, tels que les virus, les bactéries ou les parasites, soit des fractions purifiées de ces micro-organismes, soit des micro-organismes vivants dont le pouvoir pathogène a été atténué. Comme exemples de virus pouvant constituer un antigène selon la présente invention, il y a le virus de la rage, les herpès virus, tels que le virus de la maladie d'Aujeszky, les orthomixovirus tels que Influenzae, les picornavirus tels que le virus de la fièvre aphteuse ou les rétrovirus tels que les VIH. Comme exemples de micro-organisme du type bactérien pouvant constituer un antigène selon la présente invention, on peut citer E. Coli, et ceux des genres Pasteurella, Furonculosis, Vibriosis, Staphylococcus et Streptococcus. Comme d'exemples de parasites, il y a ceux des genres Trypanosoma, Plasmodium et Leishmania. On peut aussi citer les virus recombinants notamment les virus non enveloppés, tels que les adénovirus, le virus de la vaccine, le virus Canarypox, les herpès virus ou les baculovirus. On désigne aussi un vecteur recombinant viral non enveloppé vivant, dont le génome contient, insérée de préférence dans une partie non essentielle pour la réplication du virus enveloppé correspondant, une séquence codant pour une sous - unité antigénique induisant une synthèse d'anticorps et/ou un effet protecteur contre le susdit virus enveloppé ou micro-organisme pathogène ; ces sous - unités antigéniques peuvent être par exemple, une protéine, une glycoprotéine, un peptide ou une fraction peptidique et/ou protectrice contre une infection par un micro-organisme vivant tel un virus enveloppé, une bactérie ou un parasite. Le gène exogène inséré dans le micro-organisme peut être, par exemple, issu d'un virus Aujeszky ou HIV.

On peut citer notamment un plasmide recombinant constitué d'une séquence de nucléotides, dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte.

Par générateur "in vivo" d'un composé comprenant une séquence d'acides aminés, on désigne tout produit biologique capable d'exprimer ledit composé dans l'organisme hôte dans lequel on a introduit ledit générateur in vivo. Le composé comprenant la séquence d'acides aminés, peut être une protéine, un peptide ou une glycoprotéine. Ces générateurs in vivo sont généralement obtenus par des procédés issus du génie génétique. Plus particulièrement, ils peuvent consister en des micro-organismes vivants, généralement un virus, jouant le rôle de vecteur recombinant, dans lequel est insérée une séquence nucléotidique, notamment un gène exogène. Ces composés sont connus en tant que tels et utilisés notamment comme vaccin sous unitaire recombinant. A cet égard, on peut se référer à l'article de M. ELOIT et al., Journal of virology (1990) 71, 2925-2431 et aux demandes internationales de brevet publiées sous les numéros WO-A-91/00107 et WO-A-94/16681. Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par LIN et al., Circulation 82:2217,2221 ; COX et al., J. of VIROL, Sept. 1993, 67, 9, 5664-5667 et dans la demande internationale publiée sous le numéro WO 95/25542. Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune,
(ii) avoir une action curative vis-à-vis d'une maladie, essentiellement une maladie d'ordre fonctionnel, qui s'est déclenchée chez l'organisme hôte. Dans ce cas, le générateur in vivo permet un traitement de l'hôte, du type thérapie génique.

A titre d'exemple, une telle action curative peut consister en une synthèse par le générateur in vivo de cytokines, comme les interleukines, notamment l'interleukine 2. Celles-ci permettent le déclenchement ou le renforcement d'une réaction immune visant à l'élimination sélective des cellules cancéreuses.

Une composition selon l'invention comprend une concentration en antigène qui dépend de la nature de cet antigène et de la nature du sujet traité. Il est toutefois particulièrement remarquable qu'un adjuvant selon l'invention, permette de diminuer notablement la dose habituelle d'antigène requise. La concentration adéquate d'antigène peut être déterminée de manière classique par l'homme du métier. Généralement, cette dose est de l'ordre de 0,1 µg / cm³ à 1 g / cm³ plus généralement comprise entre 1 µg / cm³ et 100 mg / cm³. La concentration dudit générateur in vivo dans la composition selon l'invention dépend, là encore, notamment de la nature dudit générateur et de l'hôte dans lequel il est administré. Cette concentration peut être aisément déterminée par l'homme du métier, sur la base d'expérience de routine. A titre indicatif, on peut toutefois préciser que lorsque le générateur in vivo est un micro-organisme recombinant, sa concentration dans la composition selon l'invention peut être comprise entre 10² et 10¹⁵ microorganismes/cm³, de préférence entre 10⁵ et 10¹² microorganismes/cm³. Lorsque le générateur in vivo est un plasmide recombinant, sa concentration dans la composition selon l'invention peut être comprise entre 0,01 g /dm³ et 100 g / dm³. Le vaccin tel que défini précédemment, est préparé en mélangeant la phase adjuvante et la phase antigénique, en ajoutant éventuellement de l'eau ou un milieu diluant injectable.

La présente invention a aussi pour objet, une composition solide constituée d'un-mélange de la composition adjuvante solide telle que définie précédemment et d'un lyophilisat de phase antigénique.

La présente invention a encore pour objet, un procédé de préparation d'un vaccin, par dispersion dans un milieu aqueux injectable, d'une quantité efficace d'un mélange constitué de la composition adjuvante solide telle que définie précédemment et d'un lyophilisat de phase antigénique.

La présente invention a enfin pour objet, une forme pharmaceutique injectable constituée de deux contenants contenus dans un même emballage, caractérisée en ce que :
le premier contenant contient un mélange constitué de la composition adjuvante solide telle que définie précédemment et d'un lyophilisat de phase antigénique et en ce que :
   le deuxième contenant contient un milieu aqueux injectable.

Selon les constituants mis en oeuvre, une telle forme pharmaceutique est destinée à la vaccination humaine ou vétérinaire.

Ces derniers objets de la présente invention présentent plus particulièrement des avantages en matière de stockage et de conservation des vaccins et notamment des vaccins atténués vivants. Ils permettent aussi de diminuer le risque de contamination des sujets soumis à la vaccination grâce à la préparation dudit vaccin juste avant son injection dans le sujet.

Les exemples suivants illustrent l'invention sans toutefois la limiter.

### Exemple 1

**1 -** On prépare les compositions adjuvantes solides A₁ à A₇ suivantes, en mélangeant les ingrédients en poudres dans les proportions indiquées dans le tableau suivant (pourcentage pondéraux) :

| | A₁ | A₂ | A₃ | A₄ | A₅ | A₆ | A₇ |
|---|---|---|---|---|---|---|---|
| Lactose | 9 % | 29 % | 29 % | 14 % | 14 % | 9,5 % | 0% |
| GlMn | 89 % | 69 % | 59 % | 69 % | 69 % | 69,5% | 100% |
| CtNa | 2% | 2% | 2% | 2% | 2% | 2% | 0% |
| GlCa | 0% | 0% | 10% | 15% | 0 | 9,5% | 0% |
| LaspCa | 0% | 0% | 0% | 0% | 15 % | 9,5% | 0% |
| Total | 100 % | 100 % | 100 % | 100 % | 100 % | 100 % | 100 % |

| | | | | | | | |
|---|---|---|---|---|---|---|---|
| GlCa : gluconate de calcium ; CtNa : citrate de sodium ; LaspCa : L aspartate de calcium | | | | | | | |

**2 -** On prépare des solutions aqueuses des compositions solides A₁ à A₇, dosées à 100 mg par cm³ de gluconate de manganèse.
**3 -** On prépare les solutions injectables V₁ à V₇ en mélangeant 0,2 cm³ de chacune des solutions respectives de A₁ à C₇ avec de 1,8 cm³ de sérum et 20 µl d'une solution mère d'ovalbumine dosée à 10 mg par cm³. Les solutions ainsi formées contiennent donc 10 mg par cm³ de gluconate de manganèse et 0,1 mg. par cm³ d'ovalbumine. On prépare aussi une solution témoin T contenant 0,1 mg. par cm³ d'ovalbumine sans adjuvant.
**4 -** On prépare 8 groupes G₁ à G₈ de cinq souris femelles, souche OF1, d'un poids moyen de 18 à 20 g. On injecte 100 µl de solution V₁ à chacune des souris du groupe G₁, 100 µl de solution V₂ à chacune des souris du groupe G₂, 100µl de solution V₃ à chacune des souris du groupe G₃, 100 µl de solution V₄ à chacune des souris du groupe G₄, 100 µl de solution V₅ à chacune des souris du groupe G₅ , 100 µl de solution V₆ à chacune des souris du groupe G₆, 100 µl de solution V₇ à chacune des souris du groupe G₇ et 100 µl de solution T à chacune des souris du groupe G₈. Une vaccination de rappel est faite après 28 jours. Les prélèvements sont effectués J = 14 jours, 28 jours, 42 jours, 56 jours et 90 jours. les dosages des IgG1 et IgG2a sont réalisés par la technique ELISA.

Les résultats sont consignés dans le tableau suivant :

| | **V₁** | **V₂** | **V₃** | **V₄** | **V₅** | **V₆** | **V₇** | **T** |
|---|---|---|---|---|---|---|---|---|
| | **IgG1** | | | | | | | |
| **J = 14 j** | 400 | 800 | 2400 | 1 600 | 3200 | 800 | 2400 | 100 |
| **J = 28 j** | 600 | 3 200 | 2 400 | 1 600 | 6 400 | 1 600 | 6 400 | 100 |
| **J = 42 j** | 96 000 | 128 000 | 192 000 | 192 000 | 256 000 | 128 000 | 256 000 | 1000 |
| **J = 56 j** | 96 000 | 128 000 | 192 000 | 192 000 | 192 000 | 128 000 | 256 000 | 2000 |
| **J = 90 j** | 32 000 | 64 000 | 128 000 | 128 000 | 96 000 | 64 000 | 128 000 | 1000 |

| | **IgG2a** | | | | | | | |
|---|---|---|---|---|---|---|---|---|
| **J = 14 j** | 100 | 100 | 100 | 100 | 100 | 100 | 100 | 100 |
| **J = 28 j** | 100 | 100 | 100 | 100 | 100 | 100 | 100 | 100 |
| **J = 42 j** | 4 000 | 1 000 | 32 000 | 8000 | 1 000 | 12 000 | 8 000 | 1000 |
| **J = 56 j** | 2000 | 1 500 | 24 000 | 4 000 | 2 000 | 8 000 | 8 000 | 1000 |
| **J = 90 j** | 2 000 | 2 000 | 12 000 | 3 000 | 4 000 | 6 000 | 4 000 | 1000 |

### Exemple 2

**1 -** On prépare par simple mélange, des constituants sous agitation douce les phases grasses suivantes :

| | Φ₁ | Φ₂ | Φ₃ | Φ₄ |
|---|---|---|---|---|
| KROVOL^{™} (% en poids) | 80 | 60 | 30 | 0 |
| oléate de mannitane (% en poids) | 10 | 10 | 10 | 10 |
| oléate de mannitane éthoxylé (8 OE) (% en poids) | 10 | 30 | 60 | 90 |
| HLB | 12,7 | 12,3 | 11,7 | 11,1 |

| | | | | |
|---|---|---|---|---|
| KKOVOL^{™} : huile de maïs éthoxylée (40 OE) | | | | |

**2 -** On prépare les compositions adjuvantes solides B₁ à B₄ suivantes, en mélangeant les poudres de sels de cations multivalents et de support solide injectable dans un mélangeur Diosna^{™} LP3, en ajoutant sous agitation et en granulant, les phases grasses préparées au paragraphe précédent.
On obtient des granulés blancs, poudreux et homogènes de composition suivante (% pondéraux) :

| | B₁ | B₂ | B₃ | B₄ |
|---|---|---|---|---|
| AEROSIL^{™} 200 | 24 | 24 | 24 | 24 |
| GlMn | 6 | 6 | 6 | 6 |
| Φ₁ | 70 | 0 | 0 | 0 |
| Φ₂ | 0 | 70 | 0 | 0 |
| Φ₃ | 0 | 0 | 70 | 0 |
| Φ₄ | 0 | 0 | 0 | 70 |

| | | | | |
|---|---|---|---|---|
| AEROSIL^{™} 200 : silice pyrogénée hydrophile ; GlMn : gluconate de manganèse, | | | | |

**3 -** On prépare des solutions aqueuses des compositions solides B₁ à B₄, dosées à 100 mg par cm³ de gluconate de manganèse :
**4 -** On prépare les solutions injectables W₁ à W₄ en mélangeant 0,2 cm³ de chacune des solutions respectives de B₁ à B₄ avec de 1,8 cm³ de sérum et 20 µl d'une solution mère d'ovalbumine dosée à 10 mg par cm³. Les solutions ainsi formées contiennent donc 10 mg par cm³ de gluconate de manganèse et 0,1 mg. par cm³ d'ovalbumine. On prépare aussi une solution témoin T₁ contenant 0,1 mg. par cm³ d'ovalbumine et 10 mg par cm³ de gluconate de manganèse et aussi une solution témoin T₂. contenant 0,1 mg. par cm³ d'ovalbumine sans adjuvant.
**5 -** On prépare 6 groupes H₁ à H₆ de cinq souris femelles, souche OF1, d'un poids moyen de 18 à 20 g. On injecte 100 µl de solution W₁ à chacune des souris du groupe H₁, 100 µl de solution W₂ à chacune des souris du groupe H₂, 100µl de solution W₃ à chacune des souris du groupe H₃, 100 µl de solution W₄ à chacune des souris du groupe H₄, 100 µl de solution T₁ à chacune des souris du groupe H₅ et 100 µl de solution T₂ à chacune des souris du groupe H₆.

Une vaccination de rappel est faite après 28 jours. Les prélèvements sont effectués J = 14 jours, 28 jours, 42 jours, 56 jours et 90 jours. Les dosages des IgG1 et IgG2a sont réalisés par la technique ELISA.

Les résultats sont consignés dans le tableau suivant :

| | **W₁** | **W₂** | **W₃** | **W₄** | **T₁** | **T₂** |
|---|---|---|---|---|---|---|
| | **IgG1** | | | | | |
| **J = 14 j** | 4 000 | 2 000 | 3 000 | 4 000 | 500 | 100 |
| **J** = **28 j** | 24 000 | 8 000 | 8 000 | 8 000 | 2 000 | 100 |
| **J = 42 j** | 1 024 000 | 512 000 | 1 024 000 | 512 000 | 256 000 | 100 |
| **J = 56 j** | 256 000 | 192 000 | 256 000 | 128000 | 96 000 | 100 |
| **J = 90 j** | 256 000 | 64 000 | 128 000 | 64 000 | 48 000 | 100 |

| | **IgG2a** | | | | | |
|---|---|---|---|---|---|---|
| **J =14 j** | 100 | 100 | 100 | 100 | 100 | 100 |
| **J = 28 j** | 100 | 100 | 100 | 100 | 100 | 100 |
| **J = 42 j** | 64 000 | 48 000 | 64 000 | 24 000 | 128000 | 100 |
| **J = 56 j** | 96 000 | 48 000 | 64 000 | 24 000 | 128 000 | 100 |
| **J = 90 j** | 16 000 | 8 000 | 32 000 | 8 000 | 24 000 | 100 |

### Exemple 3

**1 -** On prépare par simple mélange des constituants sous agitation douce, les phases grasses suivantes :

| | Φ₅ | Φ₆ | Φ₇ | Φ₈ | Φ₉ | Φ₁₀ |
|---|---|---|---|---|---|---|
| KROVOL^{™} | 0 | 0 | 0 | 0 | 100 | 0 |
| MARCOL^{™} 52 | 50 | 50 | 0 | 0 | 0 | 0 |
| oléate de mannitane | 2,5 | 5 | 28 | 2,5 | 0 | 0 |
| oléate de mannitane éthoxylé (8 OE) | 47,5 | 0 | 0 | 47,5 | 0 | 100 |
| TCM | 0 | 0 | 36,35 | 0 | 0 | 0 |
| AAB2 | 0 | 0 | 0 | 50 | 0 | 0 |
| SIMULSOL^{™} 2599 | 0 | 45 | 0 | 0 | 0 | 0 |
| SIMULSOL^{™} 1292 DF | 0 | 0 | 35,65 | 0 | 0 | 0 |

KROVOL^{™} : huile de maïs éthoxylée (40 OE) ; MARCOL^{™} 52 : huile blanche minérale mélange purifié d'hydrocarbures saturés ayant un nombre moyen d'atomes de carbone autour de 19,7 ; SIMULSOL^{™} 1292DF : huile de ricin hydrogénée éthoxylée ; SIMULSOL^{™} 2599 : acide oléique éthoxylée ; AAB2™ : huile blanche minérale mélange purifié d'hydrocarbures saturés ayant un nombre moyen d'atomes de carbone autour de 23,6 ; TCM : Triglycérides à chaînes moyennes.
**2 -** On prépare les compositions adjuvantes solides C₁ à C₆ suivantes, en introduisant la poudre de gluconate de manganèse dans un mélangeur Diosna^{™} LP3, en ajoutant sous agitation et en granulant, les phases grasses préparées au paragraphe précédent. On obtient des granulés, poudreux et homogènes de composition suivante (% pondéraux) :

| | C₁ | C₂ | C₃ | C₄ | C₅ | C₆ |
|---|---|---|---|---|---|---|
| GlMn | 80 | 80 | 80 | 80 | 80 | 80 |
| Φ₅ | 20 | 0 | 0 | 0 | 0 | 0 |
| Φ₆ | 0 | 20 | 0 | 0 | 0 | 0 |
| Φ₇ | 0 | 0 | 20 | 0 | 0 | 0 |
| Φ₈ | 0 | 0 | 0 | 20 | 0 | 0 |
| Φ₉ | 0 | 0 | 0 | 0 | 20 | 0 |
| Φ₁₀ | 0 | 0 | 0 | 0 | 0 | 20 |

| | | | | | | |
|---|---|---|---|---|---|---|
| GlMn : gluconate de manganèse. | | | | | | |

**3 -** On prépare des solutions aqueuses de compositions solides C₁ à C₆ dosées à 30 mg par cm³.
**4 -** On prépare les solutions injectables X₁ à X₆ en mélangeant 1,0 cm³ de chacune des solutions respectives de C₁ à C₆ avec de 0,98 cm³ de sérum et 20 µl d'une solution mère d'ovalbumine dosée à 10 mg par cm³. Les solutions ainsi formées contiennent donc 12 mg par cm³ de gluconate de manganèse et 0,1 mg. par cm³ d'ovalbumine. On prépare aussi une solution témoin T₃ contenant 0,1 mg. par cm³ d'ovalbumine et 12 mg par cm³ de gluconate de manganèse et aussi une solution témoin T₄ contenant 0,1 mg par cm³ d'ovalbumine sans adjuvant.
**5 -** On prépare 8 groupes I₁ à I₈ de cinq souris femelles, souche OF1, d'un poids moyen de 18 à 20 g. On injecte 100 µl de solution X₁ à chacune des souris du groupe I₁, 100 µl de solution X₂ à chacune des souris du groupe I₂, 100 µl de solution X₃ à chacune des souris du groupe I₃, 100 µl de solution X₄ à chacune des souris du groupe I₄, 100 µl de solution X₅ à chacune des souris du groupe, 100 µl de solution X₆ à chacune des souris du groupe I₆,100µl de solution T₃ à chacune des souris du groupe I₇ et 100 µl de solution T₄ à chacune des souris du groupe I₈.

Une vaccination de rappel est faite après 28 jours. Les prélèvements sont effectués J = 14 jours, 28 jours, 42 jours, 56 jours et 90 jours. Les dosages des IgG1 et IgG2a sont réalisés par la technique ELISA.

Les résultats sont consignés dans le tableau suivant :

| | **X₁** | **X₂** | **X₃** | **X₄** | **X₅** | **X₆** | **T₃** | **T₄** |
|---|---|---|---|---|---|---|---|---|
| | **IgG1** | | | | | | | |
| **14 j** | 300 | 800 | 400 | 1 600 | 600 | 400 | 1 600 | 100 |
| **28 j** | 1 600 | 64 00 | 1 600 | 6 400 | 3 200 | 1 200 | 6 400 | 100 |
| **42 j** | 64 000 | 192 000 | 128 000 | 256 000 | 192 000 | 128 000 | 192 000 | 1 000 |
| **56 j** | 48 000 | 64 000 | 96 000 | 128 000 | 128 000 | 64 000 | 96 000 | 1 000 |
| **90 j** | 8 000 | 16 000 | 8000 | 32 000 | 48000 | 32 000 | 16 000 | 1 000 |

| | **IgG2a** | | | | | | | |
|---|---|---|---|---|---|---|---|---|
| **14 j** | 100 | 100 | 100 | 100 | 100 | 100 | 100 | 100 |
| **28 j** | 100 | 100 | 100 | 100 | 100 | 100 | 100 | 100 |
| **42 j** | 64000 | 4000 | 8 000 | 24000 | 12000 | 2000 | 12000 | 100 |
| **56 j** | 16 000 | 1 000 | 2 000 | 2 000 | 1 000 | 1 000 | 1 500 | 1 000 |
| **90 j** | 3 000 | 1 000 | 1 000 | 1 000 | 1 000 | 1 000 | 1 000 | 1 000 |

## Revendications

1. Composition solide adjuvante de vaccin comprenant un support solide injectable **caractérisée en ce qu'**elle se présente sous la forme de granulés.

2. Composition telle que définie à la revendication 1 **caractérisée en ce que** les granulés contiennent au plus 5% d'eau.

3. Composition telle que définie à l'une quelconque des revendications 1 ou 2 **caractérisée en ce que** le support solide injectable est choisi parmi une silice pyrogénée, un sucre injectable et plus particulièrement le lactose, le dextrose ou le mannitol, un polysaccharide et plus particulièrement une dextrine ou une cyclodextrine, un polymère cellulosique et plus particulièrement l'hydroxypropyl méthyl cellulose, la méthyl cellulose ou l'éthyl cellulose, un sel de cation métallique multivalent ou un mélange de sels de cations métalliques multivalents ou un mélange de deux ou plusieurs de ces composés.

4. Composition telle que définie à la revendication 3, **caractérisée en ce que** les cations métalliques multivalents, sont des cations métalliques divalents ou trivalents et sont plus particulièrement choisis parmi les cations divalents du calcium, du magnésium, du manganèse ou du zinc ou bien parmi les cations trivalents du fer ou de l'aluminium.

5. Composition telle que définie à l'une quelconque des revendications 3 ou 4, **caractérisée en ce que** les sels de cations métalliques sont choisis parmi les hydroxyde, carbonate, citrate, gluconate, glucoheptonate, fructoheptonate, lactate, acétate, propionate, salicylate, chlorure ou glycérophosphate.

6. Composition telle que définie à l'une quelconque des revendications 3 à 5, **caractérisée en ce que** les sels de cations métalliques sont choisis parmi l'hydroxyde de calcium, le carbonate de magnésium, le carbonate de manganèse, le gluconate de calcium, le gluconate de manganèse, le glycérophosphate de manganèse, le gluconate de zinc, le fructoheptonate de calcium, le salicylate d'aluminium ou l'acétate d'aluminium.

7. Composition telle que définie à l'une quelconque des revendications 1 à 6, **caractérisée en ce que** le support solide injectable est un sel de cation métallique multivalent ou un mélange de sels de cations métalliques multivalents et est plus particulièrement un sel de calcium ou un sel de manganèse ou un mélange de ces sels.

8. Composition telle que définie à l'une quelconque des revendications 1 à 6, **caractérisée en ce que** le support solide injectable est un mélange d'un ou plusieurs sels de cations métalliques multivalents avec un ou plusieurs sucres injectables.

9. Composition telle que définie à l'une quelconque des revendications 1 à 6, **caractérisée en ce que** le support solide injectable est de la silice pyrogénée hydrophile ou un mélange de silice pyrogénée hydrophile avec un ou plusieurs sucres injectables.

10. Composition telle que définie à l'une quelconque des revendications 1 à 6, **caractérisée en ce que** le support solide injectable est un mélange d'un ou plusieurs sels de cations métalliques multivalents et de silice pyrogénée hydrophile.

11. Composition telle que définie à l'une quelconque des revendications 1 à 10, **caractérisée en ce qu'**elle comprend en outre un agent tensioactif ou un mélange d'agents tensioactifs.

12. Composition telle que définie à la revendication 11, dans laquelle l'agent tensioactif ou le mélange d'agents tensioactifs a un nombre HLB global compris entre 5 et 15.

13. Composition telle que définie à l'une quelconque des revendications 1 à 12, **caractérisée en ce qu'**elle comprend en outre un ou plusieurs corps gras.

14. Composition telle que définie à l'une quelconque des revendications 11 à 13, **caractérisée en ce qu'**elle comprend une association comprenant :
de 50% à 95% en poids d'oléate de mannitane éthoxylé ayant un nombre d'OE compris entre 5 et 15, et, de préférence, entre 7 et 11 et
de 5 % à 50% en poids d'oléate de mannitane.

15. Composition telle que définie à l'une quelconque des revendications 11 à 14, **caractérisée en ce qu'**elle comprend entre 10 % et 90 % en poids de support solide injectable, et entre 10 % et 90 % en poids de tensioactif ou entre 10 % et 90% en poids d'un mélange constitué d'un ou plusieurs corps gras et d'un ou plusieurs tensioactifs.

16. Composition telle que définie aux revendications 9, 10 et 15, **caractérisée en ce qu'**elle comprend au plus 30 % en poids de support solide injectable.

17. Composition telle que définie aux revendications 7, 8 et 15, **caractérisée en ce qu'**elle comprend au moins 50% en poids de support solide injectable.

18. Composition telle que définie à l'une quelconque des revendications 1 à 17, **caractérisée en ce qu'**elle comprend en outre entre 1% et 10% en poids d'au moins un agent dispersant injectable.

19. Composition telle que définie à la revendication 18, **caractérisée en ce que** l'agent dispersant injectable, est choisi parmi les sels de sodium ou d'ammonium des acides citrique, glycolique, lactique, phosphorique, tartrique ou acétique.

20. Procédé de préparation de la composition telle que définie à l'une quelconque des revendications 11 à 19, comprenant les étapes suivantes :
(a) si nécessaire, la préparation d'un mélange de tous les éléments constitutifs du support solide injectable avec ajout éventuel de l'agent dispersant,
(b) l'addition sous agitation, des tensioactifs, et éventuellement des corps gras liquides, au mélange préparé à l'étape (a), ou à la poudre de support solide injectable, dans un mélangeur - granulateur pour former des granulés.

21. Utilisation de la composition telle que définie à l'une quelconque des revendications 1 à 19, comme phase adjuvante d'une composition vaccinale.

22. Procédé de préparation d'une composition injectable comprenant l'adjonction à une phase antigénique, d'une quantité efficace de la composition telle que définie à l'une quelconque des revendications 1 à 19.

23. Composition solide constituée d'un mélange de la composition adjuvante solide telle que définie à l'une quelconque des revendications 1 à 19 et d'un lyophilisat de phase antigénique.

24. Procédé de préparation d'un vaccin, par dispersion dans un milieu aqueux injectable d'un mélange constitué de la composition adjuvante solide telle que définie à l'une quelconque des revendications 1 à 19 et d'un lyophilisat de phase antigénique.

25. Forme pharmaceutique injectable, constituée de deux contenants contenus dans un même emballage, **caractérisée en ce que** le premier contenant contient un mélange constitué de la composition adjuvante solide telle que définie à l'une quelconque des revendications 1 à 19 et d'un lyophilisat de phase antigénique et **en ce que** le deuxième contenant contient un milieu aqueux injectable.

## Claims

1. Solid vaccine adjuvant composition comprising an injectable solid support **characterized in that** it is in the form of granules.

2. Composition according to Claim 1, **characterized in that** the granules contain at most 5% water.

3. Composition according to either one of Claims 1 and 2, **characterized in that** the injectable solid support is chosen from a pyrogenic silica, an injectable sugar and more particularly lactose, dextrose or mannitol, a polysaccharide and more particularly a dextrin or a cyclodextrin, a cellulose polymer and more particularly hydroxypropyl methyl cellulose, methyl cellulose or ethyl cellulose, a multivalent metal cation salt or a mixture of multivalent metal cation salts or a mixture of two or more of these compounds.

4. Composition according to Claim 3, **characterized in that** the multivalent metal cations are divalent or trivalent metal cations and are more particularly chosen from divalent cations of calcium, of magnesium, of manganese or of zinc or else from trivalent cations of iron or of aluminium.

5. Composition according to either one of Claims 3 and 4, **characterized in that** the metal cation salts are chosen from hydroxides, carbonates, citrates, gluconates, glucoheptonates, fructo-heptonates, lactates, acetates, propionates, salicylates, chlorides or glycerophosphates.

6. Composition according to any one of Claims 3 to 5, **characterized in that** the metal cation salts are chosen from calcium hydroxide, magnesium carbonate, manganese carbonate, calcium gluconate, manganese gluconate, manganese glycerophosphate, zinc gluconate, calcium fructoheptonate, aluminium salicylate or aluminium acetate.

7. Composition according to any one of Claims 1 to 6, **characterized in that** the injectable solid support is a multivalent metal cation salt or a mixture of multivalent metal cation salts and is more particularly a calcium salt or a manganese salt or a mixture of these salts.

8. Composition according to any one of Claims 1 to 6, **characterized in that** the injectable solid support is a mixture of one or more multivalent metal cation salts with one or more injectable sugars.

9. Composition according to any one of Claims 1 to 6, **characterized in that** the injectable solid support is hydrophilic pyrogenic silica or a mixture of hydrophilic pyrogenic silica with one or more injectable sugars.

10. Composition according to any one of Claims 1 to 6, **characterized in that** the injectable solid support is a mixture of one or more multivalent metal cation salts and hydrophilic pyrogenic silica.

11. Composition according to any one of Claims 1 to 10, **characterized in that** it also comprises a surfactant or a mixture of surfactants.

12. Composition according to Claim 11, in which the surfactant or the mixture of surfactants has an overall HLB number between 5 and 15.

13. Composition according to any one of Claims 1 to 12, **characterized in that** it also comprises one or more fatty substances.

14. Composition according to any one of Claims 11 to 13, **characterized in that** it comprises a combination comprising:
from 50% to 95% by weight of ethoxylated mannitan oleate having an EO number between 5 and 15 and preferably between 7 and 11; and
from 5% to 50% by weight of mannitan oleate.

15. Composition according to any one of Claims 11 to 14, **characterized in that** it comprises between 10% and 90% by weight of injectable solid support and between 10% and 90% by weight of surfactant or between 10% and 90% by weight of a mixture composed of one or more fatty substances and of one or more surfactants.

16. Composition according to Claims 9, 10 and 15, **characterized in that** it comprises at most 30% by weight of injectable solid support.

17. Composition according to Claims 7, 8 and 15, **characterized in that** it comprises at least 50% by weight of injectable solid support.

18. Composition according to any one of Claims 1 to 17, **characterized in that** it also comprises between 1% and 10% by weight of at least one injectable dispersant.

19. Composition according to Claim 18, **characterized in that** the injectable dispersant is chosen from the sodium or ammonium salts of citric, glycolic, lactic, phosphoric, tartaric or acetic acids.

20. Process for preparing the composition according to any one of Claims 11 to 19, comprising the following steps:
(a) if necessary, the preparation of a mixture of all the constituent elements of the injectable solid support with optional addition of dispersant; and
(b) the addition, with stirring, of surfactants, and optionally liquid fatty substances, to the mixture prepared in step (a) or to the injectable solid support powder, in a mixer-granulator in order to form granules.

21. Use of the composition according to any one of Claims 1 to 19, as the adjuvant phase of a vaccine composition.

22. Process for preparing an injectable composition comprising the addition to an antigenic phase of an effective amount of the composition according to any one of Claims 1 to 19.

23. Solid composition composed of a mixture of the solid adjuvant composition according to any one of Claims 1 to 19 and of an antigenic-phase lyophilisate.

24. Process for preparing a vaccine, by dispersion, in an injectable aqueous medium, of a mixture composed of the solid adjuvant composition according to any one of Claims 1 to 19 and of an antigenic-phase lyophilisate.

25. Injectable pharmaceutical form, composed of two containers contained in one and the same package, **characterized in that** the first container contains a mixture composed of the solid adjuvant composition according to any one of Claims 1 to 19 and of an antigenic-phase lyophilisate and **in that** the second container contains an injectable aqueous medium.

## Patentansprüche

1. Feste Impfstoffadjuvanszusammensetzung, die einen festen injizierbaren Träger umfaßt, **dadurch gekennzeichnet, daß** sie in Form eines Granulats vorliegt.

2. Zusammensetzung nach Anspruch 1, **dadurch gekennzeichnet, daß** das Granulat maximal 5% Wasser enthält.

3. Zusammensetzung nach einem der Ansprüche 1 oder 2, **dadurch gekennzeichnet, daß** der feste injizierbare Träger aus der folgenden Reihe stammt: pyrogene Silica; injizierbare Zucker, insbesondere Lactose, Dextrose oder Mannit; Polysaccharid, insbesondere ein Dextrin oder Cyclodextrin; celluloseartiges Polymer, insbesondere Hydroxypropylmethylcellulose, Methylcellulose oder Ethylcellulose; Salz eines mehrwertigen Metallkations oder Mischung von Salzen von mehrwertigen Metallkationen; oder Mischung von zwei oder mehr dieser Komponenten.

4. Zusammensetzung nach Anspruch 3, **dadurch gekennzeichnet, daß** es sich bei den mehrwertigen Metallkationen um zweiwertige oder dreiwertige Metallkationen handelt, insbesondere solche aus der Reihe der zweiwertigen Kationen des Calciums, des Magnesiums, des Mangans oder des Zinks, oder solche aus der Reihe der dreiwertigen Kationen des Eisens oder des Aluminiums.

5. Zusammensetzung nach einem der Ansprüche 3 oder 4, **dadurch gekennzeichnet, daß** die Salze von Metallkationen aus der folgenden Reihe stammen: Hydroxid, Carbonat, Citrat, Gluconat, Glucoheptonat, Fructoheptonat, Lactat, Acetat, Propionat, Salicylat, Chlorid oder Glycerophosphat.

6. Zusammensetzung nach einem der Ansprüche 3 bis 5, **dadurch gekennzeichnet, daß** die Salze der Metallkationen aus der folgenden Reihe stammen: Calciumhydroxid, Magnesiumcarbonat, Mangancarbonat, Calciumgluconat, Mangangluconat, Manganglycerophosphat, Zinkgluconat, Calciumfructoheptonat, Aluminiumsalicylat oder Aluminiumacetat.

7. Zusammensetzung nach einem der Ansprüche 1 bis 6, **dadurch gekennzeichnet, daß** es sich bei dem festen injizierbaren Träger um ein Salz eines mehrwertigen Metallkations oder um eine Mischung von Salzen von mehrwertigen Metallkationen, insbesondere um ein Calciumsalz oder ein Mangansalz oder eine Mischung dieser Salze handelt.

8. Zusammensetzung nach einem der Ansprüche 1 bis 6, **dadurch gekennzeichnet, daß** es sich bei dem injizierbaren festen Träger um eine Mischung von einem oder mehreren Salzen von mehrwertigen Metallkationen mit einem oder mehreren injizierbaren Zuckern handelt.

9. Zusammensetzung nach einem der Ansprüche 1 bis 6, **dadurch gekennzeichnet, daß** es sich bei dem injizierbaren festen Träger um hydrophile pyrogene Silica oder um eine Mischung von hydrophiler pyrogener Silica mit einem oder mehreren injizierbaren Zuckern handelt.

10. Zusammensetzung nach einem der Ansprüche 1 bis 6, **dadurch gekennzeichnet, daß** es sich bei dem injizierbaren festen Träger um eine Mischung von einem oder mehreren Salzen von mehrwertigen Metallkationen und hydrophiler pyrogener Silica handelt.

11. Zusammensetzung nach einem der Ansprüche 1 bis 10, **dadurch gekennzeichnet, daß** sie weiterhin ein Tensid oder eine Tensidmischung umfaßt.

12. Zusammensetzung nach Anspruch 11, bei der das Tensid oder die Tensidmischung einen Gesamt-HLB-Wert zwischen 5 und 15 aufweist.

13. Zusammensetzung nach einem der Ansprüche 1 bis 12, **dadurch gekennzeichnet, daß** sie weiterhin einen oder mehrere Fettkörper umfaßt.

14. Zusammensetzung nach einem der Ansprüche 11 bis 13, **dadurch gekennzeichnet, daß** sie eine Kombination umfassend:
50 Gew.-% bis 95 Gew.-% ethoxyliertes Mannitanoleat mit einem OE-Wert zwischen 5 und 15, vorzugsweise zwischen 7 und 11, und
5 Gew.-% bis 50 Gew.-% Mannitanoleat
umfaßt.

15. Zusammensetzung nach einem der Ansprüche 11 bis 14, **dadurch gekennzeichnet, daß** sie zwischen 10 Gew.-% und 90 Gew.-% injizierbaren festen Träger, zwischen 10 Gew.-% und 90 Gew.-% Tensid oder zwischen 10 Gew.-% und 90 Ges.-% eine Mischung bestehend aus einem oder mehreren Fettkörpern und einem oder mehreren Tensiden umfaßt.

16. Zusammensetzung nach einem der Ansprüche 9, 10 und 15, **dadurch gekennzeichnet, daß** sie maximal 30 Gew.-% eines injizierbaren festen Trägers umfaßt.

17. Zusammensetzung nach einem der Ansprüche 7, 8 und 15, **dadurch gekennzeichnet, daß** sie mindestens 50 Gew.-% eines injizierbaren festen Trägers umfaßt.

18. Zusammensetzung nach einem der Ansprüche 1 bis 7, **dadurch gekennzeichnet, daß** sie weiterhin zwischen 1 Gew.-% und 10 Gew.-% an mindestens einem injizierbaren Dispergiermittel umfaßt.

19. Zusammensetzung nach Anspruch 18, **dadurch gekennzeichnet, daß** das injizierbare Dispergiermittel aus der folgenden Reihe stammt: Natrium- oder Ammoniumsalze der Citronen-, Glykol-, Milch-, Phosphor-, Wein- oder Essigsäure.

20. Verfahren zur Herstellung der Zusammensetzung nach einem der Ansprüche 11 bis 19, umfassend die folgenden Schritte:
(a) falls erforderlich, Herstellung einer Mischung von allen Bestandteilen des injizierbaren festen Trägers, wobei gegebenenfalls das Dispergiermitttel zugesetzt wird,
(b) Zugabe der Tenside und gegebenenfalls der flüssigen Fettkörper zu der im Schritt (a) hergestellten Mischung, oder zu dem Pulver des injizierbaren festen Trägers, unter Bewegen im Mischgranulator, um ein Granulat zu bilden.

21. Verwendung der Zusammensetzung nach einem der Ansprüche 1 bis 19 als Adjuvans-Phase einer Impfstoffzusammensetzung.

22. Verfahren zur Herstellung einer injizierbaren Zusammensetzung, umfassend die Zugabe einer wirksamen Menge der Zusammensetzung nach einem der Ansprüche 1 bis 19 zu einer Antigen-Phase.

23. Feste Zusammensetzung, die aus einer Mischung der festen Adjuvanszusammensetzung nach einem der Ansprüche 1 bis 19 und einem Lyophilisat der Antigen-Phase besteht.

24. Verfahren zur Herstellung eines Impfstoffs durch Dispergieren einer Mischung, die aus der festen Adjuvanszusammensetzung nach einem der Ansprüche 1 bis 19 und einem Lyophilisat der Antigen-Phase besteht, in einem injizierbaren wäßrigen Medium.

25. Injizierbare Darreichungsform, die aus zwei Behältern, die in einer Packung enthalten sind, besteht, **dadurch gekennzeichnet, daß** der erste Behälter eine Mischung, die aus der festen Adjuvanszusammensetzung nach einem der Ansprüche 1 bis 19 und einem Lyophilisat der Antigen-Phase besteht, enthält und der zweite Behälter ein injizierbares wäßriges Medium enthält.
